# EUROPÄISCHE PATENTSCHRIFT

(11) **EP 1 292 711 B1**
(45) Veröffentlichungstag und Bekanntmachung des Hinweises auf die Patenterteilung: **30.03.2005**
(21) Anmeldenummer: 01953112.8
(22) Anmeldetag: 19.06.2001
(51) Int. Cl.: C12Q 1/68

(54) **VERFAHREN ZUR HOCHPARALLELEN ANALYSE VON POLYMORPHISMEN**
METHOD FOR THE HIGH-PARALLEL ANALYSIS OF POLYMORPHISMS
PROCEDE POUR L'ANALYSE ULTRAPARALLELE DE POLYMORPHISMES

(30) Priorität: 19.06.2000 DE 10029914
(43) Veröffentlichungstag der Anmeldung: 19.03.2003
(73) Patentinhaber: Epigenomics AG, 10435 Berlin (DE)
(72) Erfinder: BERLIN, Kurt, 14532 Stahnsdorf (DE); GUT, Ivo, Glynne, F-75014 Paris (FR)
(74) Vertreter: Schubert, Klemens, Dr.
(86) Internationale Anmeldenummer: PCT/DE2001/002273
(87) Internationale Veröffentlichungsnummer: WO 2001/098527

(56) Entgegenhaltungen:
- EP-A- 1 164 201
- WO-A-00/18960
- WO-A-01/48244
- DE-A- 10 021 204
- DE-A- 19 530 336
- US-A- 5 952 174
- US-A1- 2001 014 449
- WANG D G ET AL: "Large-scale identification, mapping, and genotyping of single-nucleotide polymorphisms in the human genome" SCIENCE, AMERICAN ASSOCIATION FOR THE ADVANCEMENT OF SCIENCE,, US, Bd. 280, 1998, Seiten 1077-1082, XP002089398 ISSN: 0036-8075
- SAUER S ET AL: "A novel procedure for efficient genotyping of single nucleotide polymorphisms" NUCLEIC ACIDS RESEARCH, OXFORD UNIVERSITY PRESS, SURREY, GB, Bd. 28, Nr. 5, 1. März 2000 (2000-03-01), Seiten E13i-e13viii, XP002158531 ISSN: 0305-1048
- FAN JIAN-BING ET AL: "Parallel genotyping of human SNPs using generic high-density oligonucleotide tag arrays." GENOME RESEARCH, Bd. 10, Nr. 6, 1. Juni 2000 (2000-06-01), Seiten 853-860, XP002934219 ISSN: 1088-9051

### SEQUENZPROTOKOLL

<110> Epigenomics AG
<120> Verfahren zur hochparallelen Analyse von Polymorphismen
<130> E01/1206/WO
<140>
   <141>
<160> 2
<170> PatentIn Ver. 2.1
<210> 1
   <211> 20
   <212> DNA
   <213> Künstliche Sequenz
<220>
   <223> Beschreibung der künstlichen Sequenz:Primer
<400> 1
   attaatatta tgtaaagtga 20
<210> 2
   <211> 20
   <212> DNA
   <213> Künstliche Sequenz
<220>
   <223> Beschreibung der künstlichen Sequenz:Primer
<400> 2
   cttacaacca ttcacccaca 20

## Beschreibung

Die vorliegende Erfindung beschreibt ein Verfahren zur hochparallelen Analyse von Polymorphismen, insbesondere SNPs. Gleichzeitig oder in einem separaten Experiment kann das Verfahren zur Analyse von DNA-Methylierung eingesetzt werden.

Das Humangenomprojekt, die Erstsequenzierung des menschlichen Genoms, wird in den nächsten Jahren abgeschlossen sein. Durch dieses Projekt wird es möglich werden, alle etwa 100.000 Gene zu identifizieren. Die Sequenzinformation öffnet ungeahnte Möglichkeiten für die Aufklärung der Genfunktionen. Dies wiederum eröffnet die Möglichkeit, Pharmakogenetik und Pharmakogenomik zu betreiben. Die Pharmakogenetik und Pharmakogenomik zielt auf den Einsatz von Medikamenten in Abhängigkeit eines Genotypen. Dadurch soll die Effektivität von Medikamenten gesteigert werden. Der notwendige Zwischenschritt ist die Bestimmung der Polymorphismen und Genotypen, die mit einem bestimmten Ansprechen assoziiert sind. Verlangt werden deshalb immer effizientere Genotypisierungsmethoden.

Derzeit gibt es zwei Kategorien von polymorphen Markern, die zur Genotypisierung eingesetzt werden, Mikrosatelliten und Single Nucleotide Polymorphisms (SNPs). Mikrosatelliten sind hoch polymorph, d.h. sie haben eine Vielzahl von Allelen. Sie sind dadurch charakterisiert, dass ein repetitives Sequenzelement, mit einer unterschiedlichen Anzahl Wiederholungen für unterschiedliche Allele, von konservierten Sequenzen flankiert ist. Durchschnittlich gibt es einen Mikrosatellitenmarker pro 1 Million Basen. Eine Karte von 5.000 positionierten Mikrosatellitenmarkern wurde von CEPH publiziert (Dil, C. et al. Nature, March 14, 1994). Mikrosatelliten werden durch die Grössenbestimmung von Produkten einer PCR mit Primern der konservierten, flankierenden Sequenz genotypisiert. Die fluoreszent markierten PCR Produkte werden auf Gelen aufgetrennt.

Es gibt vergleichsweise wenige beschriebene SNP Marker. Eine Karte mit 300.000 SNP Markern wird derzeit vom SNP Konsortium enwickelt und wird öffentlich zugänglich sein. Es gibt eine Handvoll Genotypisierungsmethoden für SNPs. Einige basieren auf der Auftrennung von Produkten auf Gelen, wie der oligonucleotide ligase assay (OLA). Er eignet sich daher eher für den mittleren Durchsatz. Andere vertrauen auf reine Hybridisierung, die jedoch nicht die gleiche Stringenz hat. DNA Arrays (DNA chips) eignen sich für die Analyse einer grossen Anzahl SNPs in einer beschränkten Anzahl von Individuen. Bis jetzt sind Beispiele gezeigt worden, in denen 1.500 SNPs auf einem DNA Chip genotypisiert wurden. Die wirkliche Stärke von DNA Chips liegt in Ansätzen, wie der Resequenzierung und der Expressionsanalyse. Ansätze, welche Primerverlängerung anwenden sind gezeigt worden. Diese haben den Vorteil, dass wenn mit fluoreszenzmarkierten Terminatorbasen gearbeitet wird, die Resultate mit einem einfachen ELISA Lesegerät gesammelt werden können.

In dem US-Patent 5,952,174 ist ein Verfahren zur Analyse von Polymorphismen mittels immobilisierter Sonden beschrieben, das mit Hilfe von Nukleasen durchgeführt werden kann. Die Nuklease wird nur eingesetzt, um die zu untersuchende doppelsträngige DNA in einzelsträngige DNA zu überführen und so eine erleichterte Bindung der DNA an die Sonden zu ermöglichen. Der Nuklease-Abbau kann jedoch weder zur Eliminierung von Hintergrundsignalen noch zur Sensivitätssteigerung eingesetzt werden.

Ein Verfahren zur Analyse von Polymorphismen mit Hilfe eines nukleolytischen Abbaus immobilisierter Sonden ist in DE 10021204 beschrieben. Auch hier wird die zu untersuchende DNA an Sonden hybridisiert, die danach sequenzspezifisch verlängert werden. Anschließend erfolgt eine Nuklease-Behandlung, um die spezifisch verlängerten Sonden zu verkürzen. Hiermit wird die nachfolgende Analyse der Sonden erleichtert, jedoch nicht die Beseitigung der unspezifischen Hintergrundsignale ermöglicht.

Ein weiteres Verfahren zur Analyse von Polymorphismen mit Hilfe immobilisierter Sonden und Nukleasen ist in EP 1164201 beschrieben.Hierbei wird die zu untersuchende DNA spezifisch an immobilisierte Oligonukleotide hybridisiert. Trägt die zu untersuchene Nukleinsäure den nachzuweisenen Polymorphismus nicht, so kommt es in dem Hybrid zu Basenfehlpaarungen. Diese Basenfehlpaarungen werden anschließend von einer besonderen Nuklease erkannt und geschnitten. Das Verfahren ist jedoch nicht in der Lage, Nukleasen unabhängig vom Vorliegen von Fehlpaarungen für den Abbau nicht oder nicht besonders verlängerter Sonden zu benutzen.

Es gibt einige SNP Genotypisierungsmethoden, die Massenspektrometrie zur Analyse verwenden. Diese haben den wesentlichen Vorteil, dass die allelspezifischen Produkte eine physische Darstellung der Produkte sind und kein fluoreszierendes Signal, dass indirekt dem Produkt zugeordnet werden muss.

Eine Methode, die kürzlich vorgestellt wurde, ist der Invader Assay und als Variante davon der Invader Squared (T. Griffin and L.M. Smith Proceedings of the ASMS 1998).

Für diese Methode werden mindestens zwei Oligonukleotide verwendet, die einen bekannten SNP abdecken. Ein Oligonukleotid deckt die Sequenz von der 5'-Seite bis unmittelbar zum SNP hin ab, so dass sich der SNP an das 3'-Ende dieses Oligonukleotids anschliesst. Meistens werden zwei weitere Oligonukleotide, von denen jeder ein Allel des Polymorphismus abdeckt und einen unterschiedliche 5'-Überhang hat, an das System hybridisiert. Eine strukturaktive Endonuklease entfernt vom vollständig komplementären Oligonukleotid den 5'-Überhang. Der abgekappte Überhang wird mittels Massenspektrometrie analysiert und zur Identifikation des Allels verwendet. Ein Nachteil der gezeigten Methode ist, dass die Produkte vor der massenspektrometrischen Analyse gründlich gereinigt werden müssen. Für diese Aufreinigung werden magnetic beads verwendet, die nicht einfach in der Handhabung sind. Dies ist ein wesentlicher Nachteil von vielen Genotypisierungsmethoden, die Massenspektrometrie zur Analyse verwenden.

Eine weitere Genotypisierungsmethode ist der Taq Man Assay. In diesem wird allelspezifisch enzymatisch ein Fluorezenzlöscher von einem fluoreszenzfarbstofftragenden Oligonukleotid getrennt.

Matrix-assisted laser desorption/ionization time-offlight Massenspektrometrie (MALDI) hat die Analytik von Biomolekülen revolutioniert (Karas, M. & Hillenkamp, F. Anal. Chem. 60, 2299-2301 (1988)). MALDI ist in verschiedenen Varianten zur Analyse von DNA eingesetzt worden. Die Varianten reichen von primer extension bis Sequenzierung (Liu, Y.-H., et al. Rapid Commun. Mass Spectrom. 9, 735-743 (1995); Ch'ang, L.-Y., et al. Rapid Commun. Mass Spectrom. 9, 772-774 (1995); Little, D.P., et al. J. Mol. Med. 75, 745-750 (1997); Haff, L. & Smirnov, I.P. Genome Res. 7, 378-388 (1997), Fei, Z., Ono, T. & Smith, L.M.

Nucleic Acids Res. 26, 2827-2828 (1998); Ross, P., Hall, L., Smirnov, I. & Haff, L. Nature Biotech. 16, 1347-1351 (1998);Ross, P.L., Lee, K. & Belgrader, P. Anal. Chem. 69, 4197-4202 (1997); Griffin, T.J., Tang, W. & Smith, L.M. Nature Biotech. 15, 1368-1372 (1997)). Der grösste Nachteil dieser Methoden ist, dass alle eine gründliche Aufreinigung der Produkte vor der MALDI Analyse bedingen. Spin column Aufreinigung oder der Einsatz von magnetic bead technology oder reversed-phase Aufreinigung sind notwendig.

Die Analyse von DNA im MALDI ist stark abhängig vom Ladungszustand des Produktes. Eine 100-fache Verbesserung der Empfindlichkeit in der MALDI Analyse kann dadurch erzielt werden, dass der Ladungszustand auf dem zu analysierenden Produkt so kontrolliert wird, dass nur eine einzige positive oder negative Überschussladung vorhanden ist. So modifizierte Produkte sind auch wesentlich weniger anfällig für die Ausbildung von Addukten (z.B. mit Na und K, Gut, I.G. and Beck, S. (1995) Nucleic Acids Res., 23, 1367-1373; Gut, I.G., Jeffery, W.A., Pappin, D.J.C. and Beck, S. Rapid Commun. Mass Spectrom., 11, 43-50 (1997)). Ein SNP Genotypisierungsverfahren, welches von diesen Bedingungen Gebrauch macht, mit dem Namen "GOOD Assay" wurde kürzlich vorgestellt (Sauer, S. et al., Nucleic Acids Research, Methods online, 2000, 28, e13). Nachteil ist, dass das gesamte Verfahren einen beschränkten Multiplexierungsgrad zulässt, und die Probenvorbereitung immer den Einsatz modernster und teurer Pipettiertechnologie bedingt.

5-Methylcytosin ist die häufigste kovalent modifizierte Base in der DNA eukaryotischer Zellen. Sie spielt beispielsweise eine Rolle in der Regulation der Transkription, beim genetischen Imprinting und in der Tumorgenese. Die Identifizierung von 5-Methylcytosin als Bestandteil genetischer Information ist daher von erheblichem Interesse. 5-Methylcytosin-Positionen können jedoch nicht durch Sequenzierung identifiziert werden, da 5-Methylcytosin das gleiche Basenpaarungsverhalten aufweist wie Cytosin. Darüber hinaus geht bei einer PCR-Amplifikation die epigenetische Information, welche die 5-Methylcytosine tragen, vollständig verloren.

Eine relativ neue und die inzwischen am häufigsten angewandte Methode zur Untersuchung von DNA auf 5-Methylcytosin beruht auf der spezifischen Reaktion von Bisulfit mit Cytosin, das nach anschließender alkalischer Hydrolyse in Uracil umgewandelt wird, welches in seinem Basenpaarungsverhalten dem Thymidin entspricht. 5-Methylcytosin wird dagegen unter diesen Bedingungen nicht modifiziert. Damit wird die ursprüngliche DNA so umgewandelt, dass Methylcytosin, welches ursprünglich durch sein Hybridisierungsverhalten vom Cytosin nicht unterschieden werden kann, jetzt durch "normale" molekularbiologische Techniken als einzig verbliebenes Cytosin beispielsweise durch Amplifikation und Hybridisierung oder Sequenzierung nachgewiesen werden kann. Alle diese Techniken beruhen auf Basenpaarung, welche jetzt voll ausgenutzt wird. Der Stand der Technik, was die Empfindlichkeit betrifft, wird durch ein Verfahren definiert, welches die zu untersuchende DNA in einer Agarose-Matrix einschließt, dadurch die Diffusion und Renaturierung der DNA (Bisulfit reagiert nur an einzelsträngiger DNA) verhindert und alle Fällungs- und Reinigungsschritte durch schnelle Dialyse ersetzt (Olek, A. et al., Nucl. Acids. Res. 1996, 24, 5064-5066). Mit dieser Methode können einzelne Zellen untersucht werden, was das Potential der Methode veranschaulicht. Allerdings werden bisher nur einzelne Regionen bis etwa 3000 Basenpaare Länge untersucht, eine globale Untersuchung von Zellen auf Tausenden von möglichen Methylierungsanalysen ist nicht möglich. Allerdings kann auch dieses Verfahren keine sehr kleinen Fragmente aus geringen Probenmengen zuverlässig analysieren. Diese gehen trotz Diffusionsschutz durch die Matrix verloren.

Eine Übersicht über die weiteren bekannten Möglichkeiten, 5-Methylcytosine nachzuweisen, kann aus dem folgenden Übersichtsartikel entnommen werden: Rein, T., DePamphilis, M. L., Zorbas, H., Nucleic Acids Res. 1998, 26, 2255.

Die Bisulfit-Technik wird bisher bis auf wenige Ausnahmen (z. B. Zechnigk, M. et al., Eur. J. Hum. Gen. 1997, 5, 94-98) nur in der Forschung angewendet. Immer aber werden kurze, spezifische Stücke eines bekannten Gens nach einer Bisulfit-Behandlung amplifiziert und entweder komplett sequenziert (Olek, A. und Walter, J., Nat. Genet. 1997, 17, 275-276) oder einzelne Cytosin-Positionen durch eine "Primer-Extension-Reaktion" (Gonzalgo, M. L. und Jones, P. A., Nucl. Acids Res. 1997, 25, 2529-2531, WO-Patent 9500669) oder einen Enzymschnitt (Xiong, Z. und Laird, P. W., Nucl. Acids. Res. 1997, 25, 2532-2534) nachgewiesen. Zudem ist auch der Nachweis durch Hybridisierung beschrieben worden (Olek et al., WO 99 28498).

Weitere Publikationen, die sich mit der Anwendung der Bisulfit-Technik zum Methylierungsnachweis bei einzelnen Genen befassen, sind: Xiong, Z. und Laird, P. W. (1997), Nucl. Acids Res. 25, 2532; Gonzalgo, M. L. und Jones, P. A. (1997), Nucl. Acids Res. 25, 2529; Grigg, S. und Clark, S. (1994), Bioassays 16, 431; Zeschnik, M. et al. (1997), Human Molecular Genetics 6, 387; Teil, R. et al. (1994), Nucl. Acids Res. 22, 695; Martin, V. et al. (1995), Gene 157, 261; WO 97 46705, WO 95 15373 und WO 45560.

Eine Übersicht über den Stand der Technik in der Oligomer Array Herstellung lässt sich aus einer im Januar 1999 erschienenen Sonderausgabe von Nature Genetics (Nature Genetics Supplement, Volume 21, January 1999), der dort zitierten Literatur und dem US-Patent 5994065 über Methoden zur Herstellung von festen Trägern für Zielmoleküle wie Oligonukleotide bei vermindertem nichtspezifischem Hintergrundsignal entnehmen.

Für die Abtastung eines immobilisierten DNA-Arrays sind vielfach fluoreszent markierte Sonden verwendet worden. Besonders geeignet für Fluoreszenzmarkierungen ist das einfache Anbringen von Cy3 und Cy5 Farbstoffen am 5'-OH der jeweiligen Sonde. Die Detektion der Fluoreszenz der hybridisierten Sonden erfolgt beispielsweise über ein Konfokalmikroskop. Die Farbstoffe Cy3 und Cy5 sind, neben vielen anderen, kommerziell erhältlich.

Genomische DNA wird durch Standardmethoden aus DNA von Zell-, Gewebe- oder sonstigen Versuchsproben gewonnen. Diese Standardmethodik findet sich in Referenzen wie Fritsch und Maniatis eds., Molecular Cloning: A Laboratory Manual, 1989.

Aufgabe der vorliegenden Erfindung ist es, ein Verfahren zur hochparallelen Analyse von Polymorphismen zur Verfügung zu stellen, welches die Nachteile des Standes der Technik überwindet.

Gegenstand der Erfindung ist ein Verfahren zur hochparallelen Charakterisierung von Polymorphismen, bei dem man die folgenden Schritte ausführt:
a. man bindet einen Satz von Sonden an eine adressierte Oberfläche,
b. man hybridisiert eine zu untersuchende Nukleinsäure an diese Sonden;
c. man verlängert die Sonden in einer allelspezifischen Reaktion, wobei diejenigen Sonden, die an eine Nukleinsäure mit dem nach nachzuweisenden Polymorphismus hybridisiert sind, unter Einbau einer blockierenden Funktion so verlängert werden, dass ein Abbau im nächsten Schritt verhindert wird, während die jenigen Sonden, an die Keine Nukleinsäusen oder an die Nukleinsäuren ohne den nachzuweisenden Polymorphismus hybridisiert sind, nicht verlängert werden oder so verlängert werden, das ein Abbau im nächsten Schritt nicht Verhindert wird;
d. man behandelt die Sonden mit einer Nuklease;
e. man analysiert die verbleibenden allelspezifischen Verlängerungsprodukte.

Erfindungsgemäß bevorzugt ist dabei, dass die Adresse der Oberfläche in Schritt a) die Position (in einem Oligonukleotidarray), eine Farbe, eine Fluoreszenzmarkierung, eine isotopische Markierung, eine chemische Markierung oder eine radioaktive Markierung ist.

Weiterhin ist erfindungsgemäß bevorzugt, dass die zu untersuchende Nukleinsäure in Schritt b) genomische DNA, mit einer Bisulfitlösung vorbehandelte DNA, klonierte DNA, cDNA, RNA, ein PCR Produkt oder ein Ligationsprodukt ist.

Erfindungsgemäß bevorzugt ist auch, dass man die Sonden in Abhängigkeit von der jeweiligen Sequenz des daran hybridisierten Templats mittels einer Polymerase und modifizierten Nukleotidbausteinen zu spezifischen Produkten entsprechend Schritt c) umsetzt.

Bevorzugt ist ferner, dass man die Sonden in Abhängigkeit von der jeweiligen Sequenz des daran hybridisierten Templats mittels einer Ligase und einem phosphorylierten Oligonukleotid zu spezifischen Verlängerungsprodukten, entsprechend Schritt c) umsetzt.

Weiterhin ist erfindungsgemäß bevorzugt, dass die Verlängerungsreaktion oder die Art der Verlängerungsreaktion von einem SNP (Single Nucleotide Polymorphism) in der Proben DNA abhängt.

Besonders ist es erfindungsgemäß bevorzugt, dass die zu untersuchende Nukleinsäure eine mit einer Bisulfitlösung (=Hydrogensulfit, Disulfit) vorbehandelte genomische DNA Probe ist und dass die Verlängerungsreaktion oder die Art der Verlängerungsreaktion vom Methylierungsstatus von Cytosin-Basen in der genomischen DNA-Probe abhängt.

Ganz besonders ist ein erfindungsgemäßes Verfahren bevorzugt, bei dem man SNPs und DNA-Methylierung gleichzeitig untersucht.

Bevorzugt ist erfindungsgemäß, dass man in der Verlängerungsreaktion wenigstens ein Nukleotid anfügt, welches von einer 3'-Exonuklease nicht oder nur mit erheblich verminderter Effizienz abspaltbar ist. In einigen Fällen ist besonders bevorzugt, dass dieses Nukleotid ein Methylphosphonat, Phosphorothioat, Phosphorodithioat, Methylphosphorothioat, ein alkyliertes Phosphorothioat oder -Dithioat oder ein Derivat dieser Verbindungen ist.

Weiterhin ist erfindungsgemäß bevorzugt, dass man am betreffenden Nukleotidbaustein entweder an der Nukleobase oder an der Desoxyribose einen Substituenten anfügt, welcher den Abbau durch eine 3'-Exonuklease verhindert.

Bevorzugt ist ferner, dass man in Schritt d) eine 3'-Exonuklease verwendet. Dabei ist insbesondere erfindungsgemäß bevorzugt, dass man als 3'-Exonuklease Phosphodiesterase aus Crotalus durissus (snake venom phosphodiesterase), Escherichia coli polymerase I, II, oder III, T4 DNA Polymerase, T7 DNA Polymerase (unmodifiziert), Phosphodiesterase II Typ I-SA oder Calf Thymus 54 kDa Polypeptid mit 3'- Exonuclease Aktivität verwendet.

Bevorzugt ist erfindungsgemäß auch, dass man die Verlängerungsprodukte für die Detektion mit einer nachweisbaren Markierung versieht. Bevorzugt ist ebenso, dass man komplementäre Oligomere an die Verlängerungsprodukte hybridisiert, die für die Detektion mit einer nachweisbaren Markierung versehen sind. Hierbei ist besonders bevorzugt, dass die komplementären Oligomere Oligonukleotide, RNA-Oligomere oder PNA-Oligomere (Peptide Nucleic Acids) sind. Dabei ist weiterhin ganz besonders bevorzugt, dass die Markierungen Fluoreszenzmarkierungen sind und/oder dass die Markierungen Radionuklide sind und/oder dass die Markierungen ablösbare Massenmarkierungen sind, die in einem Massenspektrometer nachgewiesen werden und/oder dass man die Verlängerungsprodukte oder die komplementären Oligomere selbst über ihre Masse in einem Massenspektrometer nachweist.

Erfindungsgemäß bevorzugt ist aber auch, dass man die allelspezifischen Verlängerungsprodukte mittels Massenspektrometrie analysiert und/oder dass man Fragmente der allelspezifischen Verlägerungsprodukte mittels Massenspektrometrie analysiert. Besonders bevorzugt ist dabei auch noch, dass man Matrix-assistierte Laser Desorptions/Ionisations Massenspektrometrie (MALDI) oder Elektrospray Ionisations Massenspektrometrie (ESI) zur Analyse verwendet.

Hierzu ist es erfindungsgemäß vorteilhaft, dass die Sonden oder die komplementären Oligomere in einer Art vorliegen, die sich besonders gut zur massenspektrometrischen Analyse eignen. Bevorzugt ist dabei, dass die besonders gute Eignung zur massenspektrometrischen Analyse dadurch zustande kommt, dass die allelspezifischen Produkte netto einfach positiv oder einfach negativ geladen sind.

Weiterhin ist erfindungsgemäß bevorzugt, dass auf einem adressierten Analysepunkt der Oberfläche eine Vielzahl von unterschiedlichen Sonden sind.

Bevorzugt ist erfindungsgemäß auch, dass man bekannte Polymorphismen in der zu untersuchenden DNA genotypisiert und/oder dass man unbekannte Polymorphismen in der zu untersuchenden DNA identifiziert und/oder dass man Cytosin-Methylierungen detektiert und visualisiert. Dabei ist besonders bevorzugt, dass man bekannte Methylierungsmuster in der zu analysierenden Probe untersucht.

Besonders bevorzugt ist bei dem erfindungsgemäßen Verfahren, dass man die genomische DNA aus einer DNA-Probe erhält, wobei Quellen für DNA z. B. Zelllinien, Blut, Sputum, Stuhl, Urin, Gehirn-Rückenmarks-Flüssigkeit, in Paraffin eingebettetes Gewebe, beispielsweise Gewebe von Augen, Darm, Niere, Hirn, Herz, Prostata, Lunge, Brust oder Leber, histologische Objektträger und alle möglichen Kombinationen hiervon umfassen.

Ein weiterer Gegenstand der vorliegenden Erfindung ist die Verwendung des erfindungsgemäßen Verfahrens zur Diagnose und/oder Prognose nachteiliger Ereignisse für Patienten oder Individuen, wobei diese nachteiligen Ereignisse mindestens einer folgenden Kategorien angehören: unerwünschte Arzneimittelwirkungen; Krebserkrankungen; CNS-Fehlfunktionen, Schäden oder Krankheit; Aggressionssymptome oder Verhaltensstörungen; klinische, psychologische und soziale Konsequenzen von Gehirnschädigungen; psychotische Störungen und Persönlichkeitsstörungen; Demenz und/oder assoziierte Syndrome; kardiovaskuläre Krankheit, Fehlfunktion und Schädigung; Fehlfunktion, Schädigung oder Krankheit des gastrointestinalen Traktes; Fehlfunktion, Schädigung oder Krankheit des Atmungssystems; Verletzung, Entzündung, Infektion, Immunität und/oder Rekonvaleszenz; Fehlfunktion, Schädigung oder Krankheit des Körpers als Abweichung im Entwicklungsprozess; Fehlfunktion, Schädigung oder Krankheit der Haut, der Muskeln, des Bindegewebes oder der Knochen; endokrine und metabolische Fehlfunktion, Schädigung oder Krankheit; Kopfschmerzen oder sexuelle Fehlfunktion.

Weiterhin ist ein Gegenstand der vorliegenden Erfindung die Verwendung des erfindungsgemäßen Verfahrens zur Unterscheidung von Zelltypen oder Geweben oder zur Untersuchung der Zelldifferenzierung.

Schließlich ist ein Gegenstand der vorliegenden Erfindung ein Kit, enthaltend mindestens ein Primerpaar zur Amplifikation, einen Satz von Sonden und Enzyme und Puffer und eine Anleitung zur Durchführung des erfindungsgemäßen Verfahrens.

Zur Verfügung gestellt wird ein Verfahren zur hochparallelen Genotypisierung von Polymorphismen. Dieses Verfahren übertrifft die Effizienz bestehender Verfahren, in Bezug auf die Einfachkeit der Handhabung, die Kosten, die Qualität und den Durchsatz, bei weitem. Es ist zudem für die gleichzeitige Detektion von Cytosin-Methylierung in Nukleinsäureproben geeignet.

Die Erfindung beschreibt somit ein Verfahren zur hochparallelen Charakterisierung von Polymorphismen.

Im ersten Schritt des Verfahrens wird ein Satz von Sonden an eine adressierte Oberfläche gebunden.

Man setzt als Sonden vorzugsweise Oligonukleotide, modifizierte Oligonukleotide, peptide nucleic acids (PNAs), Chimäre dieser Verbindungsklassen oder andere Substanzen ein, die sequenzspezifisch mit DNA wechselwirken.

Die jeweilige Sonde ist mit einer charakteristischen nachweisbaren Markierung versehen. In einer besonders bevorzugten Variante des Verfahrens ist die Adressierung der Oberfläche die Position in einem Oligonukleotidarray, eine Farbe, eine Fluoreszenzmarkierung, eine isotopische Markierung, eine chemische Markierung oder eine radioaktive Markierung.

Im zweiten Verfahrensschritt hybridisiert man die zu untersuchende Nukleinsäure, die vorzugsweise aus genomischer DNA, klonierter DNA, chemisch vorbehandelter DNA, cDNA, RNA, PCR Produkten oder Ligationsprodukten besteht, an die besagten Sonden.

Bevorzugt stammen die zu untersuchenden Nukleinsäuren aus einer DNA-Probe, wobei Quellen für DNA z. B. Zelllinien, Blut, Sputum, Stuhl, Urin, Gehirn-Rückenmarks-Flüssigkeit, in Paraffin einbettetes Gewebe, beispielsweise Gewebe von Augen, Darm, Niere, Hirn, Herz, Prostata, Lunge, Brust oder Leber, histologische Objektträger und alle möglichen Kombinationen hiervon umfassen.

In einer besonders bevorzugten Variante des Verfahrens wird die DNA zuvor mit einer Bisulfitlösung (Disulfit, Hydrogensulfit) behandelt.

Im dritten Verfahrensschritt verlängert man die Sonden in einer allelspezifischen enzymatischen Reaktion, welche von der Sequenz der als Templat fungierenden zu untersuchenden Nukleinsäure abhängt.

In einer besonders bevorzugten Variante des Verfahrens werden die Sonden in Abhängigkeit von der jeweiligen Sequenz des daran hybridisierten Templats mittels einer Polymerase und Nukleotidbausteinen zu spezifischen Produkten umgesetzt.

In einer wiederum bevorzugten Variante des Verfahrens werden die Sonden in Abhängigkeit von der jeweiligen Sequenz des daran hybridisierten Templats mittels einer Ligase und einem 5'-phosphorylierten Oligonukleotid zu spezifischen Produkten umgesetzt.

In einer besonders bevorzugten Variante des Verfahrens werden Methylierungsmuster in der zu analysierenden vorbehandelten DNA untersucht, und die Art oder das Stattfinden der Verlängerungsreaktion oder Ligasereaktion hängen von der Ausprägung einer potentiell methylierten Position in der zu untersuchenden Nukleinsäureprobe ab.
In diesem Fall ist es erforderlich, die Nukleinsäureprobe mit einer Bisulfitlösung vorzubehandeln, wobei nach alkalischer Hydrolyse die nicht methylierten Cytosinbasen als Uracil, die 5-Methylcytosinbasen jedoch unverändert vorliegen.

In einer weiteren besonders bevorzugten Variante des Verfahrens werden SNPs in der zu analysierenden vorbehandelten DNA untersucht, und die Art oder das Stattfinden der Verlängerungsreaktion oder Ligasereaktion hängen von der Ausprägung eines SNP (Single Nucleotide Polymorphism) in der zu untersuchenden Nukleinsäureprobe ab.

In einer besonders bevorzugten Variante des Verfahrens werden Cytosin-Methylierung und SNPs einer Nukleinsäureprobe in einem Experiment untersucht.

Auf einem adressierten Analysepunkt der Oberfläche befinden sich vorzugsweise eine Vielzahl von unterschiedlichen Sonden.

In einer besonders bevorzugten Ausführung des Verfahrens wird in der Verlängerungsreaktion zumindest ein Nukleotid oder eine andere Einheit angefügt, welche von einer 3'-Exonuklease entweder nicht oder nur mit erheblich verminderter Effizienz abgespalten werden kann. Damit wird der Abbau solcher Verlängerungsprodukte verhindert, welche charakteristisch für jeweils eine bestimmte Ausprägung eines SNPs oder einer Methylierungsposition sind.

Bevorzugt sind solche Einheiten Methylphosphonate, Phosphorothioate, Phosphorodithioate, Methylphosphorothioate, alkylierte Phosphorodithioate oder -Thioate, oder aber Derivate dieser Verbindungen sind.

Bevorzugt sind solche Einheiten auch Nukleotidbausteine, die entweder an der Nukleobase oder aber an der Desoxyribose Substituenten tragen, welche den Abbau durch eine 3'-Exonuklease verhindern.

Bevorzugt ist auch im Falle der Ligasereaktion das Anfügen eines Oligonukleotids, das zumindest eine der oben genannten chemischen Einheiten enthält.

Im vierten Verfahrensschritt werden die nicht verlängerten Sonden abgebaut. Bevorzugt werden zuvor die hybridisierten, zu untersuchenden Nukleinsäuren entfernt.

Es ist auch möglich, das Verfahren so auszuführen, dass zudem auch bestimmte Verlängerungsprodukte abgebaut werden, die nicht einer bestimmten Ausprägung eines SNPs oder einer Methylierungsposition entsprechen oder diesen nicht zuzuordnen sind.

Besonders bevorzugt erfolgt der Abbau durch eine 3'-Exonuklease, wiederum besonders bevorzugt durch Phosphodiesterase aus Crotalus durissus (snake venom phosphodiesterase).

Bevorzugt ist auch die Verwendung von Escherichia coli polymerase I, II, und III, T4 DNA Polymerase, T7 DNA Polymerase (unmodifiziert), Phosphodiesterase II Typ I-SA oder Calf Thymus 54 kDa Polypeptid mit 3'- Exonuklease Aktivität.

Im fünften Verfahrensschritt analysiert man die verbleibenden allelspezifischen Produkte.

Bevorzugt sind die Verlängerungsprodukte mit einer nachweisbaren Markierung versehen.

In einer besonders bevorzugten Variante des Verfahrens werden komplementäre Oligomere an die Verlängerungsprodukte hybridisiert. Die komplementären Oligomere sind besonders bevorzugt mit einer nachweisbaren Markierung versehen.

Besonders bevorzugt sind die komplementären Oligomere Oligonukleotide, modifizierte Oligonukleotide, peptide nucleic acids (PNAs), Chimäre dieser Verbindungsklassen oder andere Substanzen, die sequenzspezifisch mit DNA wechselwirken.

In einer besonders bevorzugten Variante des Verfahrens sind die nachweisbaren Markierungen Fluoreszenzmarkierungen.

In einer besonders bevorzugten Variante des Verfahrens sind die nachweisbaren Markierungen Radionuklide.

In einer besonders bevorzugten Variante des Verfahrens sind die nachweisbaren Markierungen ablösbare Massenmarkierungen, die in einem Massenspektrometer nachgewiesen werden.

In einer weiteren, besonders bevorzugten Variante des Verfahrens werden die Verlängerungsprodukte oder die komplementären Oligomere selbst über ihre Masse in einem Massenspektrometer nachgewiesen.

In einer besonders bevorzugten Variante des Verfahrens werden die allelspezifischen Verlängerungsprodukte mittels Massenspektrometrie analysiert. In einer weiteren, besonders bevorzugten Variante werden Fragmente der allelspezifischen Verlängerungsprodukte mittels Massenspektrometrie analysiert.

Besonders bevorzugt werden Matrix-assistierte Laser Desorptions/Ionisations Massenspektrometrie (MALDI) oder Elektrospray Ionisations Massenspektrometrie (ESI) zur Analyse verwendet.

In einer besonders bevorzugten Ausführung des Verfahrens liegen die Sonden oder die komplementären Oligomere derart vor, dass sie sich besonders gut zur massenspektrometrischen Analyse eignen. Die besonders gute Eignung zur massenspektrometrischen Analyse kommt bevorzugt dadurch zustande, dass die allelspezifischen Produkte netto einfach positiv oder einfach negativ geladen sind.

Bevorzugt befinden sich an einem adressierten Analysepunkt der Oberfläche eine Vielzahl von unterschiedlichen Sonden.

Besonders bevorzugt werden nach dem erfindungsgemäßen Verfahren bekannte Polymorphismen in der zu untersuchenden DNA genotypisiert.

Bevorzugt werden entsprechend dem erfindungsgemäßen Verfahren unbekannte Polymorphismen in der zu untersuchenden DNA genotypisiert.

Besonders bevorzugt werden entsprechend dem erfindungsgemäßen Verfahren Cytosin-Methylierungen detektiert und visualisiert.

Besonders bevorzugt werden entsprechend dem erfindungsgemäßen Verfahren bekannte Methylierungsmuster in der zu analysierenden Probe untersucht.

Gegenstand der Erfindung ist zudem die Verwendung des oben beschriebenen Verfahrens zur Diagnose und/oder Prognose nachteiliger Ereignisse für Patienten oder Individuen, wobei diese nachteiligen Ereignisse mindestens einer folgenden Kategorien angehören: unerwünschte Arzneimittelwirkungen; Krebserkrankungen; CNS-Fehlfunktionen, Schäden oder Krankheit; Aggressionssymptome oder Verhaltensstörungen; klinische, psychologische und soziale Konsequenzen von Gehirnschädigungen; psychotische Störungen und Persönlichkeitsstörungen; Demenz und/oder assoziierte Syndrome; kardiovaskuläre Krankheit, Fehlfunktion und Schädigung; Fehlfunktion, Schädigung oder Krankheit des gastrointestinalen Traktes; Fehlfunktion, Schädigung oder Krankheit des Atmungssystems; Verletzung, Entzündung, Infektion, Immunität und/oder Rekonvaleszenz; Fehlfunktion, Schädigung oder Krankheit des Körpers als Abweichung im Entwicklungsprozess; Fehlfunktion, Schädigung oder Krankheit der Haut, der Muskeln, des Bindegewebes oder der Knochen; endokrine und metabolische Fehlfunktion, Schädigung oder Krankheit; Kopfschmerzen oder sexuelle Fehlfunktion.

Gegenstand der Erfindung ist zudem die Verwendung des oben beschriebenen Verfahrens zur Unterscheidung von Zelltypen oder Geweben oder zur Untersuchung der Zelldifferenzierung.

Gegenstand der vorliegenden Erfindung ist zudem ein Kit, welcher ein Primerpaar zur Amplifikation, einen Satz von Sonden und Enzyme und Puffer und eine Anleitung zur Durchführung des oben beschriebenen Verfahrens enthält.

Das Verfahren wird abschließend durch eine Abbildung erläutert.

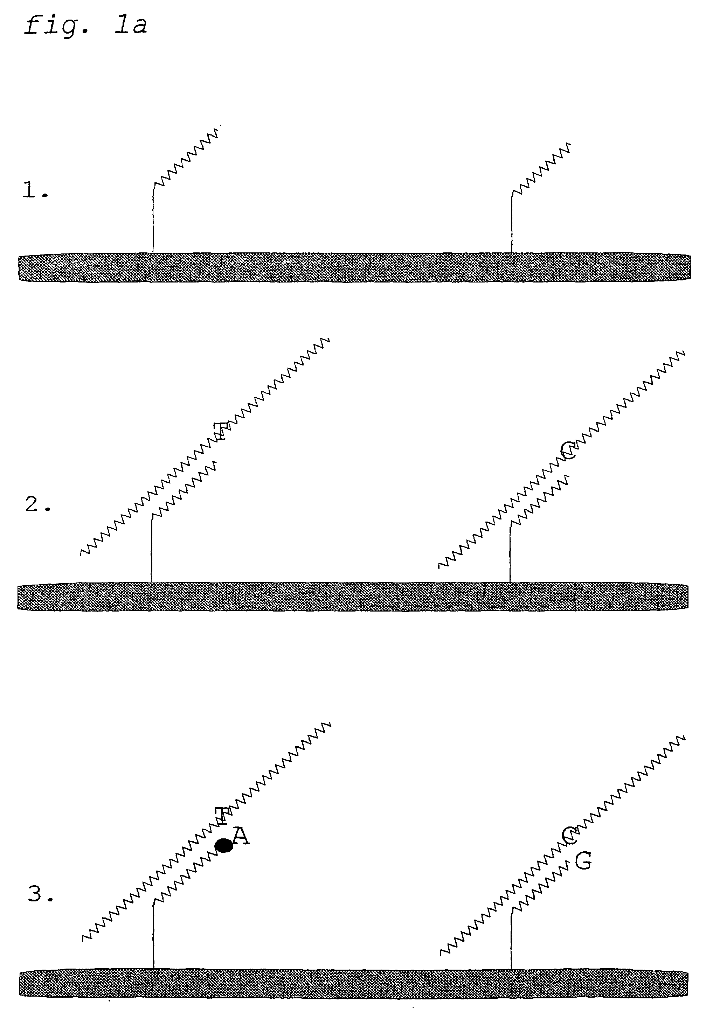

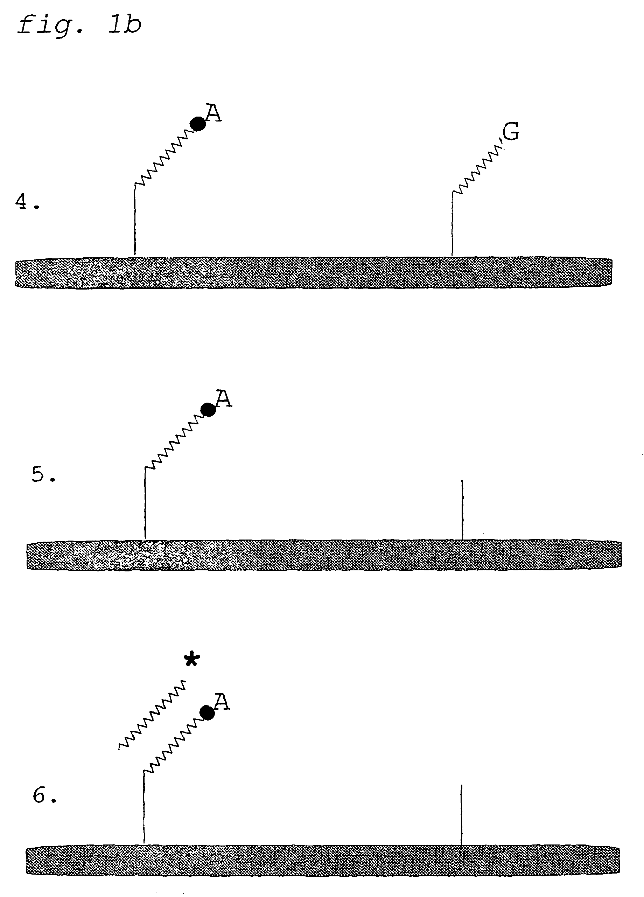

Fig. 1a und 1b veranschaulichen die Verfahrensschritte an einem Beispiel:
1. Zuerst werden Sonden an die adressierte Oberfläche gebunden.
2. Dann hybridisiert man die zu untersuchende Nukleinsäure an die Sonde.
3. Daraufhin verlängert man die Sonden in einer allelspezifischen Reaktion. Nur an den Positionen, an denen ein T vorliegt, wird mit dem Adeninbaustein eine Einheit, beispielsweise ein Phosphorothioat (in der Figur ein schwarzer Kreis), eingebaut, die den Abbau im folgenden Schritt verhindert.
4. Die zu untersuchende Nukleinsäure wird entfernt.
5. Nachfolgend werden die Sonden enzymatisch abgebaut, die im dritten Schritt nicht mit einer blockierenden Funktion versehen wurden.
6. Die verbleibenden Verlängerungsprodukte werden analysiert, indem beispielsweise ein komplementäres Oligonukleotid, welches eine Fluoreszenzmarkierung (in der Figur mit * gekennzeichnet) trägt, an die verbleibenden Verlängerungsprodukte hybridisiert wird.

Die folgenden Beispiele erläutern die Erfindung:

### Beispiel 1:

### Durchführung der Extension von immobilisierten Primeroligonukleotiden mit fluoreszenzmarkierten Nukleotiden

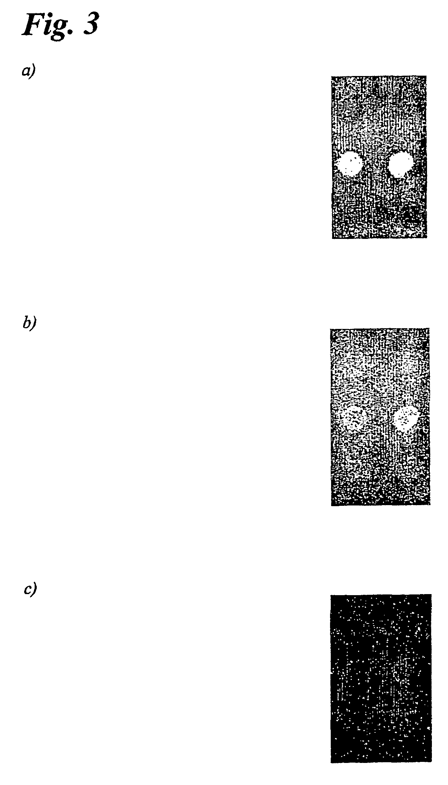

Im ersten Schritt wird eine genomische Sequenz unter Verwendung von Bisulfit (Hydrogensulfit, Disulfit) derart behandelt, dass alle nicht an der 5-Position der Base methylierten Cytosine so verändert werden, dass eine hinsichtlich dem Basenpaarungsverhalten unterschiedliche Base entsteht, während die in 5-Position methylierten Cytosine unverändert bleiben. Wird für die Reaktion Bisulfit verwendet, so findet an den nicht methylierten Cytosinbasen eine Addition statt. Zudem müssen ein denaturierendes Reagenz oder Lösungsmittel sowie ein Radikalfänger zugegen sein. Eine anschließende alkalische Hydrolyse führt dann zur Umwandlung von nicht methylierten Cytosin-Nukleobasen in Uracil. Diese umgewandelte DNA dient dazu, methylierte Cytosine nachzuweisen. Im zweiten Verfahrensschritt verdünnt man die behandelte DNA-Probe mit Wasser oder einer wässrigen Lösung. Bevorzugt wird anschließend eine Desulfonierung der DNA durchgeführt. Im dritten Schritt des Verfahrens amplifiziert man die DNA-Probe in einer Polymerasekettenreaktion, bevorzugt mit einer hitzebeständigen DNA-Polymerase. Im vorliegenden Fall werden Cytosine des Gens DAPK1 untersucht. Dazu wird mit den spezifischen Primeroligonukleotiden ATTAATATTATGTAAAGTGA (SEQ-ID:1) und CTTACAACCATTCACCCACA (SEQ-ID:2) ein definiertes Fragment der Länge 465 bp amplifiziert. Diese Amplifikat dient als Templat, welches zum Verlängern der immobilisierten Primeroligonukleotide dient. Nach Hybridisierung des Templats gegen die immobilisierten Primeroligonukleotide werden diese in einer Extensionsreaktion unter Verwendung eines Nukleotidgemisches aus Deoxynukleotiden (hier: dCTP, dTTP, dATP) und Cyanine-5 (Cy5) bzw. Cyanine-3 (Cy3) markierten Deoxynukleotiden (hier: Cy5-dCTP, Cy3-dUTP) verlängert. In Figur 3a) werden die Primeroligonukleotide nach einer Hybrisierung mit einem Cy5-fluoreszenzmarkierten Amplifikat des Gens DAPK1 und anschließender Extensionsreaktion mit Cy3- und Cy5-fluoreszenzmarkierten Nukleotiden bei einer für den Fluoreszenzfarbstoff Cy3 spezifischen Wellenlänge von 532nm detektiert. Figur 3b) zeigt die durch die bei der Extensionsreaktion eingebauten fluoreszenzmarkierten Nukleotide nach anschließender Dehybridisierung des Cy5-fluoreszenzmarkierten Amplifikats bei einer Wellenlänge von 532nm detektierten Signale. Figur 3c) dient als Kontrolle; hier können nach Hybridisierung mit dem Cy5-fluoreszenzmarkierten Amplifikat bei einer Wellenlänge von 532nm keine Signale detektiert werden.

### Beispiel 2:

### Durchführung der Extension von immobilisierten Primeroligonukleotiden mit 5'-Phosphothioat modifizierten Nukleotiden

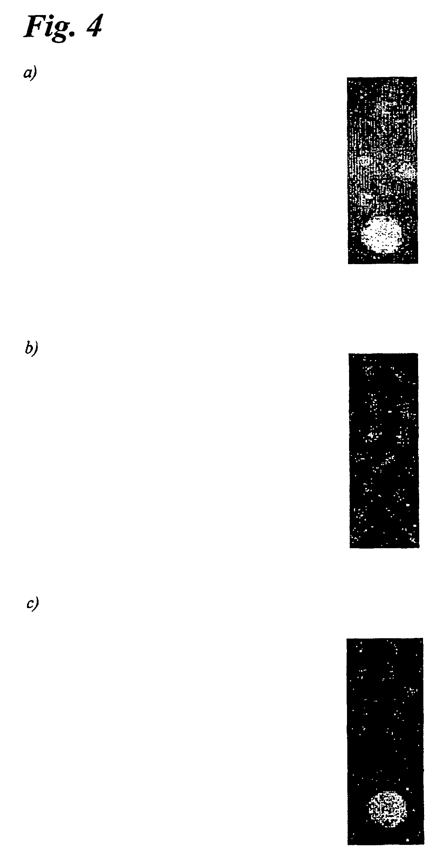

Im ersten Schritt wird eine genomische Sequenz unter Verwendung von Bisulfit (Hydrogensulfit, Disulfit) derart behandelt, dass alle nicht an der 5-Position der Base methylierten Cytosine so verändert werden, dass eine hinsichtlich dem Basenpaarungsverhalten unterschiedliche Base entsteht, während die in 5-Position methylierten Cytosine unverändert bleiben. Wird für die Reaktion Bisulfit verwendet, so findet an den nicht methylierten Cytosinbasen eine Addition statt. Zudem müssen ein denaturierendes Reagenz oder Lösungsmittel sowie ein Radikalfänger zugegen sein. Eine anschließende alkalische Hydrolyse führt dann zur Umwandlung von nicht methylierten Cytosin-Nukleobasen in Uracil. Diese umgewandelte DNA dient dazu, methylierte Cytosine nachzuweisen. Im zweiten Verfahrensschritt verdünnt man die behandelte DNA-Probe mit Wasser oder einer wässrigen Lösung. Bevorzugt wird anschließend eine Desulfonierung der DNA durchgeführt. Im dritten Schritt des Verfahrens amplifiziert man die DNA-Probe in einer Polymerasekettenreaktion, bevorzugt mit einer hitzebeständigen DNA-Polymerase. Im vorliegenden Fall werden Cytosine des Gens DAPK1 untersucht. Dazu wird mit den spezifischen Primeroligonukleotiden ATTAATATTATGTAAAGTGA (SEQ-ID:1) und CTTACAACCATTCACCCACA (SEQ-ID:2) ein definiertes Fragment der Länge 465 bp amplifiziert. Diese Amplifikat dient als Templat, welches zum Verlängern der immobilisierten Primeroligonukleotide dient. Nach Hybridisierung des Templats gegen die immobilisierten Primeroligonukleotide werden diese in einer Extensionsreaktion unter Verwendung eines Nukleotidgemisches aus Deoxynukleotiden (hier: dCTP, dTTP, dATP) und 5'-Phosphothioat modifizierten Deoxynukleotiden (hier: α-SdCTP, α-S-dUTP) verlängert. In Figur 4a) werden die Primeroligonukleotide nach Hybridisierung mit einem Cy5-fluoreszenzmarkierten Amplifikat des Gens DAPK1 und anschliessender Extensionsreaktion mit 5'-Phosphothioat modifizierten Nukleotiden bei einer für für den Fluoreszenzfarbstoff Cy5 spezifischen Wellenlänge von 635nm detektiert. Figur 4b) zeigt die durch die bei der Extensionsreaktion eingebauten fluoreszenzmarkierten Nukleotide nach anschließender Dehybridisierung des Cy5-fluoreszenzmarkierten Amplifikats bei einer Wellenlänge von 653nm detektierten Signale. In einem anschließenden Schritt werden durch Zugabe des Enzyms Phosphodiesterase 1 (PDE 1), welches DNA vom 3' Terminus ausgehend hydrolysiert, alle Primeroligonukleotide hydrolysiert, die nicht durch den Einbau eines 5'-Phosphothioat modifizierten Nukleotids geschützt sind. In Figur 4c) können bei den Primeroligonukleotiden, die durch den Einbau eines 5'-Phophothioat modifizierten Nukleotids vor der Hydrolyse des Enzyms PDE 1 geschützt waren, nach Hybridisierung mit dem Cy5-fluoreszenzmarkierten Amplifikat des Gens DAPK1 Signale bei einer Wellenlänge von 653nm detektiert werden.

## Patentansprüche

1. Verfahren zur hochparallelen Charakterisierung von Polymorphismen, **dadurch gekennzeichnet, dass** man die folgenden Schritte ausführt:
a. man bindet einen Satz von Sonden an eine adressierte Oberfläche,
b. man hybridisiert eine zu untersuchende Nukleinsäure an diese Sonden;
c. man verlängert die Sonden in einer allelspezifischen Reaktion, wobei diejenigen Sonden, die an eine Nukleinsäure mit dem nachzuweisenden Polymorphismus hybridisiert sind, unter Einbau einer blockierenden Funktion so verlängert werden, dass ein Abbau im nächsten Schritt verhindert wird, während diejenigen Sonden, an die keine Nukleinsäuren oder an die Nukleinsäuren ohne den nachzuweisenden Polymorphismus hybridisiert sind, nicht verlängert werden oder so verlängert dass ein Abbau im nächsten Schritt nicht verhindert wird.
d. man behandelt die Sonden mit einer Nuklease;
e. man analysiert die verbleibenden allelspezifischen Verlängerungsprodukte.

2. Verfahren nach Anspruch 1, **dadurch gekennzeichnet, dass** die Adresse der Oberfläche in Schritt a) die Position in einem Oligonukleotidarray, eine Farbe, eine Fluoreszenzmarkierung, eine isotopische Markierung, eine chemische Markierung oder eine radioaktive Markierung ist.

3. Verfahren nach einem der voranstehenden Ansprüche, **dadurch gekennzeichnet, dass** die zu untersuchende Nukleinsäure in Schritt b) genomische DNA, mit einer Bisulfitlösung vorbehandelte DNA, klonierte DNA, cDNA, RNA, ein PCR Produkt oder ein Ligationsprodukt ist.

4. Verfahren nach einem der voranstehenden Ansprüche, **dadurch gekennzeichnet, dass** man die Sonden in Abhängigkeit von der jeweiligen Sequenz des daran hybridisierten Templats mittels einer Polymerase und modifizierten Nukleotidbausteinen zu spezifischen Produkten entsprechend Schritt c) umsetzt.

5. Verfahren nach einem der Ansprüche 1 bis 4, **dadurch gekennzeichnet, dass** man die Sonden in Abhängigkeit von der jeweiligen Sequenz des daran hybridisierten Templats mittels einer Ligase und einem phosphorylierten Oligonukleotid zu spezifischen Verlängerungsprodukten, entsprechend Schritt c) umsetzt.

6. verfahren nach einem der voranstehenden Ansprüche, **dadurch gekennzeichnet, dass** die Verlängerungsreaktion oder die Art der Verlängerungsreaktion von einem SNP (Single Nucleotide Polymorphism) in der Proben DNA abhängt.

7. Verfahren nach einem der voranstehenden Ansprüche, **dadurch gekennzeichnet, dass** die zu untersuchende Nukleinsäure eine mit einer Bisulfitlösung vorbehandelte genomische DNA Probe ist und dass die Verlängerungsreaktion oder die Art der Verlängerungsreaktion vom Methylierungsstatus von Cytosin-Basen in der genomischen DNA-Probe abhängt.

8. Verfahren nach einem der voranstehenden Ansprüche, **dadurch gekennzeichnet, dass** man SNPs und DNA-Methylierung gleichzeitig untersucht.

9. Verfahren nach einem der voranstehenden Ansprüche, **dadurch gekennzeichnet, dass** man in der Verlängerungsreaktion wenigstens ein Nukleotid anfügt, welches von einer 3'-Exonuklease nicht oder nur mit erheblich verminderter Effizienz abspaltbar ist.

10. Verfahren nach Anspruch 1 oder 9, **dadurch gekennzeichnet, dass** dieses Nukleotid ein Methylphosphonat, Phosphorothioat, Phosphorodithioat, Methylphosphorothioat, ein alkyliertes Phosphorothioat oder - Dithioat oder ein Derivat dieser Verbindungen ist.

11. Verfahren nach einem der voranstehenden Ansprüche **dadurch gekennzeichnet, dass** man am betreffenden Nukleotidbaustein entweder an der Nukleobase oder an der Desoxyribose einen Substituenten anfügt, welcher den Abbau durch eine 3'-Exonuklease verhindert.

12. Verfahren nach einem der voranstehenden Ansprüche, **dadurch gekennzeichnet, dass** man in Schritt d) eine 3'-Exonuklease verwendet.

13. Verfahren nach Anspruch 12, **dadurch gekennzeichnet, dass** man als 3'-Exonuklease Phosphodiesterase aus Crotalus durissus (snake venom phosphodiesterase), Escherichia coli polymerase I, II, oder III, T4 DNA Polymerase, T7 DNA Polymerase unmodifiziert, Phosphodiesterase II Typ I-SA oder Calf Thymus 54 kDa Polypeptid mit 3'- Exonuclease Aktivität verwendet.

14. Verfahren nach einem der voranstehenden Ansprüche, **dadurch gekennzeichnet, dass** man die Verlängerungsprodukte für die Detektion mit einer nachweisbaren Markierung versieht.

15. Verfahren nach einem der Ansprüche 1 bis 13, **dadurch gekennzeichnet, dass** man komplementäre Oligomere an die Verlängerungsprodukte hybridisiert, die für die Detektion mit einer nachweisbaren Markierung versehen sind.

16. Verfahren nach Anspruch 15, **dadurch gekennzeichnet, dass** die komplementären Oligomere Oligonukleotide, RNA-Oligomere oder PNA-Oligomere (Peptide Nucleic Acids) sind.

17. Verfahren nach einem der Ansprüche 14 bis 16, **dadurch gekennzeichnet, dass** die Markierungen Fluoreszenzmarkierungen sind.

18. Verfahren nach einem der Ansprüche 14 bis 16, **dadurch gekennzeichnet, dass** die Markierungen Radionuklide sind.

19. Verfahren nach einem der Ansprüche 14 bis 16, **dadurch gekennzeichnet, dass** die Markierungen ablösbare Massenmarkierungen sind, die in einem Massenspektrometer nachgewiesen werden.

20. Verfahren nach einem der Ansprüche 14 bis 16, **dadurch gekennzeichnet, dass** man die Verlängerungsprodukte oder die komplementären Oligomere selbst über ihre Masse in einem Massenspektrometer nachweist.

21. Verfahren nach einem der Ansprüche 1 bis 14, **dadurch gekennzeichnet, dass** man die allelspezifischen Verlängerungsprodukte mittels Massenspektrometrie analysiert.

22. Verfahren nach einem der Ansprüche 1 bis 14, **dadurch gekennzeichnet, dass** man Fragmente der allelspezifischen Verlängerungsprodukte mittels Massenspektrometrie analysiert.

23. Verfahren nach einem der Ansprüche 19 bis 22, **dadurch gekennzeichnet, dass** man Matrix-assistierte Laser Desorptions/Ionisations Massenspektrometrie (MALDI) oder Elektrospray Ionisations Massenspektrometrie (ESI) zur Analyse verwendet.

24. Verfahren nach Anspruch 15 oder 16, **dadurch gekennzeichnet, dass** die Sonden oder die komplementären Oligomere in einer Art vorliegen, die sich besonders gut zur massenspektrometrischen Analyse eignen.

25. Verfahren nach Anspruch 24, **dadurch gekennzeichnet, dass** die besonders gute Eignung zur massenspektrometrischen Analyse dadurch zustande kommt, dass die allelspezifischen Produkte netto einfach positiv oder einfach negativ geladen sind.

26. Verfahren nach einem der voranstehenden Ansprüche, **dadurch gekennzeichnet, dass** auf einem adressierten Analysepunkt der Oberfläche eine Vielzahl von unterschiedlichen Sonden sind.

27. Verfahren nach einem der voranstehenden Ansprüche **dadurch gekennzeichnet, dass** man bekannte Polymorphismen in der zu untersuchenden DNA genotypisiert.

28. Verfahren nach einem der Ansprüche 1 bis 26, **dadurch gekennzeichnet, dass** man unbekannte Polymorphismen in der zu untersuchenden DNA identifiziert.

29. Verfahren nach einem der voranstehenden Ansprüche **dadurch gekennzeichnet, dass** man Cytosin-Methylierungen detektiert und visualisiert.

30. Verfahren nach Anspruch 27 **dadurch gekennzeichnet, dass** man bekannte Methylierungsmuster in der zu analysierenden Probe untersucht.

31. Verfahren nach einem der voranstehenden Ansprüche, wobei man die genomische DNA aus einer DNA-Probe erhält, wobei Quellen für DNA z. B. Zelllinien, Blut, Sputum, Stuhl, Urin, Gehirn-Rückenmarks-Flüssigkeit, in Paraffin eingebettetes Gewebe, beispielsweise Gewebe von Augen, Darm, Niere, Hirn, Herz, Prostata, Lunge, Brust oder Leber, histologische Objektträger und alle möglichen Kombinationen hiervon umfassen.

32. Verwendung eines Verfahrens nach einem der voranstehenden Ansprüche zur Diagnose und/oder Prognose nachteiliger Ereignisse für Patienten oder Individuen, wobei diese nachteiligen Ereignisse mindestens einer folgenden Kategorien angehören: unerwünschte Arzneimittelwirkungen; Krebserkrankungen; CNS-Fehlfunktionen, Schäden oder Krankheit; Aggressionssymptome oder Verhaltensstörungen; klinische, psychologische und soziale Konsequenzen von Gehirnschädigungen; psychotische Störungen und Persönlichkeitsstörungen; Demenz und/oder assoziierte Syndrome; kardiovaskuläre Krankheit, Fehlfunktion und Schädigung; Fehlfunktion, Schädigung oder Krankheit des gastrointestinalen Traktes; Fehlfunktion, Schädigung oder Krankheit des Atmungssystems; Verletzung, Entzündung, Infektion, Immunität und/oder Rekonvaleszenz; Fehlfunktion, Schädigung oder Krankheit des Körpers als Abweichung im Entwicklungsprozess; Fehlfunktion, Schädigung oder Krankheit der Haut, der Muskeln, des Bindegewebes oder der Knochen; endokrine und metabolische Fehlfunktion, Schädigung oder Krankheit; Kopfschmerzen oder sexuelle Fehlfunktion.

33. Verwendung eines Verfahrens nach einem der voranstehenden Ansprüche zur Unterscheidung von Zelltypen oder Geweben oder zur Untersuchung der Zelldifferenzierung.

## Claims

1. A method for the highly parallel characterization of polymorphisms, hereby **characterized in that** the following steps are conducted:
a. a set of probes is bound to an addressed surface,
b. a nucleic acid to be investigated is hybridized to these probes;
c. the probes are extended in an allele-specific reaction,
wherein those probes, which are hybridized to a nucleic acid carrying the polymorphism to be detected, are extended by incorporating a blocking function in a way that a decomposition is prevented in the next step,
while those probes which are not hybridised to nucleic acids or which are hybridized to nucleic acids not carrying the polymorphism to be detected are not extended or are extended in a way that a decomposition is not prevented in the next step;
d. the probes are treated with a nuclease;
e. the remaining allele-specific extension products are analyzed.

2. The method according to claim 1, further **characterized in that** the address of the surface in step a) is the position in an oligonucleotide array, a color, a fluorescent label, an isotopic label, a chemical label or a radioactive label.

3. The method according to one of the preceding claims, further **characterized in that** the nucleic acid to be investigated in step b) is genomic DNA, DNA pretreated with a bisulfite solution, cloned DNA, cDNA, RNA, a PCR product or a ligation product.

4. The method according to one of the preceding claims, further **characterized in that** the probes are converted to specific products, corresponding to step c), as a function of the respective sequence of the template hybridized thereon, by means of a polymerase and modified nucleotide building blocks.

5. The method according to one of claims 1 to 4, further **characterized in that** the probes are converted to specific extension products corresponding to step c), as a function of the respective sequence of the template hybridized thereon, by means of a ligase and a phosphorylated oligonucleotide.

6. The method according to one of the preceding claims, further **characterized in that** the extension reaction or the type of extension reaction depends on an SNP (Single Nucleotide Polymorphism) in the sample DNA.

7. The method according to one of the preceding claims, further **characterized in that** the nucleic acid to be investigated is a genomic DNA sample pretreated with a bisulfite solution and that the extension reaction or the type of extension reaction depends on the methylation state of cytosine bases in the genomic DNA sample.

8. The method according to one of the preceding claims, further **characterized in that** SNPs and DNA methylation are investigated simultaneously.

9. The method according to one of the preceding claims, further **characterized in that** at least one nucleotide is attached in the extension reaction, which cannot be cleaved by a 3'-exonuclease or can be cleaved only with considerably reduced efficiency.

10. The method according to claim 1 or 9, further **characterized in that** this nucleotide is a methyl phosphonate, a phosphorothioate, a phosphorodithioate, a methyl phosphorothioate, an alkylated phosphorothioate or phosphorodithioate or a derivative of these compounds.

11. The method according to one of the preceding claims, further **characterized in that** a substituent which hinders decomposition by a 3'-exonuclease is attached to the concerned nucleotide base either on the nucleobase itself or on the deoxyribose.

12. The method according to one of the preceding claims, further **characterized in that** a 3'-exonuclease is used in step d).

13. The method according to claim 12, further **characterized in that** phosphodiesterase from Crotalus durissus (snake venom phosphodiesterase), Escherichia coli polymerase I, II, or III, T4 DNA polymerase, T7 DNA polymerase unmodified, phosphodiesterase II type I-SA or calf thymus 54-kDa polypeptide with 3'-exonuclease activity is used as the 3'- exonuclease.

14. The method according to one of the preceding claims, further **characterized in that** the extension products are provided with a detectable label for detection.

15. The method according to one of claims 1 to 13, further **characterized in that** complementary oligomers are hybridized to the extension products, which are provided with a detectable label for detection.

16. The method according to claim 15, further **characterized in that** the complementary oligomers are oligonucleotides, RNA oligomers or PNA oligomers (Peptide Nucleic Acids).

17. The method according to one of claims 14 to 16, further **characterized in that** the labels are fluorescent labels.

18. The method according to one of claims 14 to 16, further **characterized in that** the labels are radionuclides.

19. The method according to one of claims 14 to 16, further **characterized in that** the labels are detachable mass labels, which are detected in a mass spectrometer.

20. The method according to one of claims 14 to 16, further **characterized in that** the extension products or the complementary oligomers themselves are detected by their mass in a mass spectrometer.

21. The method according to one of claims 1 to 14, further **characterized in that** the allele-specific extension products are analyzed by means of mass spectrometry.

22. The method according to one of claims 1 to 14, further **characterized in that** fragments of allele-specific extension products are analyzed by means of mass spectrometry.

23. The method according to one of claims 19 to 22, further **characterized in that** matrix-assisted laser desorption/ionization mass spectrometry (MALDI) or electrospray ionization mass spectrometry (ESI) is used for analysis

24. The method according to claim 15 or 16, further **characterized in that** the probes or the complementary oligomers are present in a form which is particularly well suitable for mass-spectrometric analysis.

25. The method according to claim 24, further **characterized in that** particularly good suitability for mass-spectrometric analysis is achieved if the allele-specific products have a net single positive charge or single negative charge.

26. The method according to one of the preceding claims, further **characterized in that** a plurality of different probes are [present] on one addressed analysis point of the surface.

27. The method according to one of the preceding claims, further **characterized in that** known polymorphisms in the DNA to be investigated are genotyped.

28. The method according to one of claims 1 to 26, further **characterized in that** unknown polymorphisms in the DNA to be investigated are identified.

29. The method according to one of the preceding claims, further **characterized in that** cytosine methylations are detected and visualized.

30. The method according to claim 27, further **characterized in that** known methylation patterns are investigated in the sample to be analyzed.

31. The method according to one of the preceding claims, wherein the genomic DNA is obtained from a DNA sample, whereby sources for DNA include, e.g., cell lines, blood, sputum, stool, urine, cerebrospinal fluid, tissue embedded in paraffin, for example, tissue from eyes, intestine, kidney, brain, heart, prostate, lungs, breast or liver, histological microscope slides and all possible combinations thereof.

32. Use of a method according to one of the preceding claims, for the diagnosis and/or prognosis of adverse events for patients or individuals, whereby these adverse events belong to at least one of the following categories: undesired drug interactions; cancer disorders; CNS malfunctions, damage or disease; symptoms of aggression or behavioral disturbances; clinical, psychological and social consequences of brain damage; psychotic disturbances and personality disorders; dementia and/or associated syndromes; cardiovascular disease, malfunction and damage; malfunction, damage or disease of the gastrointestinal tract; malfunction, damage or disease of the respiratory system; lesion, inflammation, infection, immunity and/or convalescence; malfunction, damage or disease of the body as an abnormality in the development process; malfunction, damage or disorder of the skin, the muscles, the connective tissue or the bones; endocrine and metabolic malfunction, damage or disorder; headaches or sexual malfunction.

33. Use of a method according to one of the preceding claims for distinguishing cell types or tissues or for investigating cell differentiation.

## Revendications

1. Procédé pour la caractérisation ultraparallèle de polymorphismes, **caractérisé en ce qu'**on effectue les étapes suivantes :
a. on fixe une série des sondes à une surface adressée ;
b. on fait hybrider avec ces sondes un acide nucléique à étudier ;
c. on prolonge les sondes dans une réaction spécifique d'allèle, les sondes qui sont hybridées avec un acide nucléique présentant le polymorphisme à détecter étant prolongées avec incorporation d'une fonction bloquante, ce qui empêche une dégradation dans l'étape suivante, tandis que les sondes avec lesquelles aucun acide nucléique n'est hybridé ou avec lesquelles sont hybridés des acides nucléiques sans le polymorphisme à détecter n'étant pas prolongées ou étant prolongées de telle façon qu'une dégradation n'est pas évitée dans l'étape suivante ;
d. on traite les sondes par une nucléase ;
e. on analyse les produits d'extension restants, spécifiques d'allèle.

2. Procédé selon la revendication 1, **caractérisé en ce que** l'adresse de la surface dans l'étape a) est la position dans un ensemble d'oligonucléotides, une couleur, un marquage fluorescent, un marquage isotopique, un marquage chimique ou un marquage radioactif.

3. Procédé selon l'une quelconque des revendications précédentes, **caractérisé en ce que** l'acide nucléique à étudier dans l'étape b) est un ADN génomique, un ADN prétraité par une solution de bisulfite, un ADN cloné, un ADNc, un ARN, un produit de PCR ou un produit de ligature.

4. Procédé selon l'une quelconque des revendications précédentes, **caractérisé en ce qu'**on fait réagir selon l'étape c) les sondes en fonction de la séquence respective de la matrice hybridée avec celles-ci, au moyen d'une polymérase et de composants nucléotidiques modifiés, pour aboutir à des produits spécifiques.

5. Procédé selon l'une quelconque des revendications 1 à 4, **caractérisé en ce qu'**on fait réagir selon l'étape c) les sondes en fonction de la séquence respective de la matrice hybridée avec celles-ci, au moyen d'une ligase et d'un oligonucléotide phosphorylé, pour aboutir à des produits d'extension spécifiques.

6. Procédé selon l'une quelconque des revendications précédentes, **caractérisé en ce que** la réaction d'extension ou la nature de la réaction d'extension dépend d'un SNP (polymorphisme d'un seul nucléotide) dans l'ADN des sondes.

7. Procédé selon l'une quelconque des revendications précédentes, **caractérisé en ce que** l'acide nucléique à étudier est un ADN génomique prétraité par une solution de bisulfite et **en ce que** la réaction d'extension ou la nature de la réaction d'extension dépend de l'état de méthylation de bases cytosine dans la sonde d'ADN génomique.

8. Procédé selon l'une quelconque des revendications précédentes, **caractérisé en ce qu'**on étudie simultanément des SNP et la méthylation d'ADN.

9. Procédé selon l'une quelconque des revendications précédentes, **caractérisé en ce qu'**on ajoute dans la réaction d'extension au moins un nucléotide qui ne peut pas être coupé par une 3'-exonucléase ou ne peut l'être qu'avec une efficacité très réduite.

10. Procédé selon la revendication 1 ou 9, **caractérisé en ce que** ce nucléotide est un méthylphosphonate, phosphorothioate, phosphorodithioate, méthylphosphorothioate, un phosphorothioate ou -dithioate alkylé ou un dérivé de ces composés.

11. Procédé selon l'une quelconque des revendications précédentes, **caractérisé en ce qu'**on ajoute au composant nucléotidique concerné, soit au niveau de la base nucléique, soit au niveau du désoxyribose, un substituant qui empêche la dégradation par une 3'-exonucléase.

12. Procédé selon l'une quelconque des revendications précédentes, **caractérisé en ce qu'**on utilise dans l'étape d) une 3'-exonucléase.

13. Procédé selon la revendication 12, **caractérisé en ce qu'**on utilise comme 3'-exonucléase la phosphodiestérase de *Crotalus durissus* (phosphodiestérase de venin de serpent), la polymérase I, II ou III d'*Escherichia coli*, l'ADN polymérase de T4, l'ADN polymérase de T7, non modifiée, la phosphodiestérase II de type I-SA ou le polypeptide de 54 kDa de thymus de veau à activité 3'-exonucléase.

14. Procédé selon l'une quelconque des revendications précédentes, **caractérisé en ce que**, pour la détection, on munit les produits d'extension d'un marquage détectable.

15. Procédé selon l'une quelconque des revendications 1 à 13, **caractérisé en ce qu'**on fait hybrider des oligomères complémentaires avec les produits d'extension qui sont munis d'un marquage détectable pour la détection.

16. Procédé selon la revendication 15, **caractérisé en ce que** les oligomères complémentaires sont des oligonucléotides, des oligomères d'ARN ou des oligomères de PNA (peptide-acides nucléiques).

17. Procédé selon l'une quelconque des revendications 14 à 16, **caractérisé en ce que** les marquages sont des marquages fluorescents.

18. Procédé selon l'une quelconque des revendications 14 à 16, **caractérisé en ce que** les marquages sont des radionuclides.

19. Procédé selon l'une quelconque des revendications 14 à 16, **caractérisé en ce que** les marquages sont des marquages massiques détachables qui sont détectés dans un spectromètre de masse.

20. Procédé selon l'une quelconque des revendications 14 à 16, **caractérisé en ce qu'**on détecte par leur masse, dans un spectromètre de masse, les produits d'extension ou les oligomères complémentaires eux-mêmes.

21. Procédé selon l'une quelconque des revendications 14 à 16, **caractérisé en ce qu'**on analyse par spectrométrie de masse les produits d'extension spécifiques d'allèle.

22. Procédé selon l'une quelconque des revendications 14 à 16, **caractérisé en ce qu'**on analyse par spectrométrie de masse des fragments des produits d'extension spécifiques d'allèle.

23. Procédé selon l'une quelconque des revendications 19 à 22, **caractérisé en ce qu'**on utilise pour l'analyse la spectrométrie de masse à désorption/ionisation laser assistée par matrice (MALDI) ou la spectrométrie de masse à ionisation par pulvérisation électrique (ESI).

24. Procédé selon la revendication 15 ou 16, **caractérisé en ce que** les sondes ou les oligomères complémentaires se trouvent en un type qui les qualifie particulièrement bien pour l'analyse par spectrométrie de masse.

25. Procédé selon la revendication 24, **caractérisé en ce que** la particulièrement bonne qualification pour l'analyse par spectrométrie de masse est due au fait que les produits spécifiques d'allèle ont une seule charge nette positive ou une seule charge nette négative.

26. Procédé selon l'une quelconque des revendications précédentes, **caractérisé en ce qu'**une multiplicité de sondes différentes se trouve sur un point d'analyse adressé de la surface.

27. Procédé selon l'une quelconque des revendications précédentes, **caractérisé en ce qu'**on détermine le génotype de polymorphismes connus dans l'ADN à étudier.

28. Procédé selon l'une quelconque des revendications 1 à 26, **caractérisé en ce qu'**on identifie des polymorphismes inconnus dans l'ADN à étudier.

29. Procédé selon l'une quelconque des revendications précédentes, **caractérisé en ce qu'**on détecte et visualise des méthylations de cytosine.

30. Procédé selon la revendication 29, **caractérisé en ce qu'**on étudie des profils connus de méthylation dans l'échantillon à analyser.

31. Procédé selon l'une quelconque des revendications précédentes, dans lequel on obtient l'ADN génomique à partir d'un échantillon d'ADN, des sources de l'ADN comprenant par exemple des lignées cellulaires, du sang, un crachat, des fèces, de l'urine, du liquide céphalo-rachidien, un tissu inclus dans de la paraffine, par exemple un tissu d'oeil, d'intestin, de rein, de cerveau, de coeur, de prostate, de poumon, de sein ou de foie, des porte-objet histologiques et toutes combinaisons possibles de ceux-ci.

32. Utilisation d'un procédé selon l'une quelconque des revendications précédentes pour le diagnostic et/ou le pronostic d'évènements défavorables pour des patients ou des individus, ces évènements défavorables appartenant à au moins l'une des catégories suivantes : effets indésirables de médicaments ; maladies cancéreuses ; dysfonctionnements, lésions ou maladie du système nerveux central (SNC) ; symptômes d'agression ou troubles du comportement ; conséquences cliniques, psychologiques et sociales de lésions cérébrales ; troubles psychotiques et troubles de la personnalité ; démence et/ou syndromes associés ; maladie, dysfonctionnement et lésion cardiovasculaire ; dysfonctionnement, lésion ou maladie du tractus gastro-intestinal ; dysfonctionnement, lésion ou maladie du système respiratoire ; blessure, inflammation, infection, immunité et/ou reconvalescence ; dysfonctionnement, lésion ou maladie du corps en tant que déviation dans le processus de développement ; dysfonctionnement, lésion ou maladie de la peau, des muscles, du tissu conjonctif ou des os ; dysfonctionnement, lésion ou maladie endocriniens et métaboliques ; céphalées ou dysfonctionnement sexuel.

33. Utilisation d'un procédé selon l'une quelconque des revendications précédentes pour l'étude de tissus ou de types cellulaires ou pour l'étude de la différentiation cellulaire.
